(19)

Europäisches
Patentamt
European
Patent Office
Office européen
des brevets

(11) **EP 3 786 179 A1**

(12) **EUROPEAN PATENT APPLICATION**
published in accordance with Art. 153(4) EPC

(43) Date of publication:
**03.03.2021 Bulletin 2021/09**

(21) Application number: **19792637.1**

(22) Date of filing: **07.05.2019**

(51) Int Cl.:
**C07K 14/78** (2006.01)     **C12N 5/071** (2010.01)

(86) International application number:
**PCT/JP2019/018272**

(87) International publication number:
**WO 2019/208832 (31.10.2019 Gazette 2019/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.04.2018   JP 2018087326**

(71) Applicants:
• **Toppan Printing Co., Ltd.
Tokyo 110-0016 (JP)**

• **Osaka University
Suita-shi, Osaka 565-0871 (JP)**

(72) Inventors:
• **KITANO Shiro
Tokyo 110-0016 (JP)**
• **IRIE Shinji
Tokyo 110-0016 (JP)**
• **MATSUSAKI Michiya
Suita-shi, Osaka 565-0871 (JP)**

(74) Representative: **TBK
Bavariaring 4-6
80336 München (DE)**

(54) **EXTRACELLULAR-MATRIX-CONTAINING COMPOSITION, METHOD FOR PRODUCING SAME, THREE-DIMENSIONAL TISSUE CONSTRUCT, AND THREE-DIMENSIONAL TISSUE CONSTRUCT FORMATION AGENT**

(57)     The present invention relates to an extracellular-matrix-containing composition comprising: a fragmented extracellular matrix component, wherein at least a part of the fragmented extracellular matrix component is crosslinked.

## Description

### Technical Field

The present invention relates to an extracellular-matrix-containing composition, a method for producing the extracellular-matrix-containing composition, a three-dimensional tissue construct, and a three-dimensional tissue construct formation agent.

### Background Art

Techniques to construct a three-dimensional tissue construct of cells ex vivo have been developed in recent years. Proposed are, for example, a method of producing a three-dimensional tissue construct by culturing coated cells, which are cultured cells whose whole surfaces are each covered with an adhesion film (Patent Literature 1), and a method of producing a three-dimensional tissue construct by seeding cells on a scaffold made of polylactic acid or the like (Non Patent Literature 1). The present inventors have previously proposed, for example, a method of producing a three-dimensional tissue construct, comprising: forming a three-dimensional tissue construct by three-dimensionally disposing cells each coated with a coating containing an extracellular matrix component such as a collagen component and a fibronectin component (Patent Literature 2), and a method of producing a three-dimensional tissue construct, comprising: forming coated cells with a coating formed on the surface of each cell; and three-dimensionally disposing the coated cells, wherein forming coated cells comprises: soaking cells in a solution containing a coating component; and separating the soaked cells and the solution containing the coating component by using a liquid-permeable membrane (Patent Literature 3). Such three-dimensional tissue constructs are expected to be applicable to alternatives for experimental animals, materials for transplantation, and so forth.

Various techniques have been examined, such as techniques using a scaffold material that allows cells to adhere thereto, and techniques of stacking cells without use of a scaffold material, and culture of cells under coexistence with an extracellular matrix component such as a collagen component is commonly performed in any of the cell culture techniques. This is because such an extracellular matrix component functions as a material to physically support the tissue structure in the intercellular matrix, and is in addition inferred to play a biologically important role in cell development, differentiation, morphogenesis, and so forth.

### Citation List

### Patent Literature

Patent Literature 1: Japanese Unexamined Patent Publication No. 2012-115254
Patent Literature 2: International Publication No. WO 2015/072164
Patent Literature 3: International Publication No. WO 2016/027853

### Non Patent Literature

Non Patent Literature 1: Nature Biotechnology, 2005, Vol. 23, NO. 7, p. 879-884
Non Patent Literature 2: Acta Biomaterialia, 2015, Vol.25, p.131-142

### Summary of Invention

### Problems to be Solved by the Invention

Extracellular matrix components are generally insoluble, and it is difficult to dissolve a large amount of an extracellular matrix component in an aqueous solution. For this reason, thickness of tissues that can be formed and the mass content ratio of an extracellular matrix component in a tissue are restricted in methods of forming a three-dimensional tissue construct by suspending a solution containing an extracellular matrix component and cells.

In view of this, the present inventors suspended a fragmented extracellular matrix component in an aqueous medium to prepare a dispersion containing a higher concentration of an extracellular matrix component, and suspended cells in the dispersion, thereby finding that tissue constructs having a thickness and a mass content ratio of an extracellular matrix component that conventional techniques fail to achieve are successfully formed.

While tissue formation materials containing a fragmented extracellular matrix component and techniques of tissue formation using such a tissue formation material are effective, it is difficult to disperse a fragmented extracellular matrix component in an aqueous medium after dry storage. Therefore, prior preparation is required for fragmented extracellular matrix components, and this is a cause of increased workloads.

The present invention was made in view of the above circumstances, and an object of the present invention is to provide an extracellular-matrix-containing composition excellent in dispersibility after dry storage, a method for producing the extracellular-matrix-containing composition, and a three-dimensional tissue construct and three-dimensional tissue construct formation agent each comprising the extracellular-matrix-containing composition.

**Means for Solving the Problems**

The present inventors diligently studied to achieve a novel object of improving the dispersibility after dry storage to find that the object is successfully achieved through the invention shown below.

Specifically, the present invention provides, for example, [1] to [13] in the following.

[1] An extracellular-matrix-containing composition comprising:

a fragmented extracellular matrix component, wherein
at least a part of the fragmented extracellular matrix component is crosslinked.

[2] The extracellular-matrix-containing composition according to (1), wherein at least a part of the fragmented extracellular matrix component is fibrillar.

[3] The extracellular-matrix-containing composition according to (1) or (2), wherein the extracellular-matrix-containing composition is dispersible in an aqueous medium.

[4] The extracellular-matrix-containing composition according to any one of (1) to (3), wherein an average length of the fragmented extracellular matrix component is 100 nm to 400 $\mu$m.

[5] The extracellular-matrix-containing composition according to any one of (1) to (4), wherein the fragmented extracellular matrix component comprises a fragmented collagen component.

[6] The extracellular-matrix-containing composition according to any one of (1) to (5), wherein a crosslinking percentage as determined with a TNBS method is 2% or more.

[7] The extracellular-matrix-containing composition according to any one of (1) to (6), wherein the extracellular-matrix-containing composition is powdery.

[8] A three-dimensional tissue construct formation agent, comprising the extracellular-matrix-containing composition according to any one of (1) to (7).

[9] A three-dimensional tissue construct comprising:

the extracellular-matrix-containing composition according to any one of (1) to (7); and
a cell.

[10] A method for producing an extracellular-matrix-containing composition comprising a fragmented extracellular matrix component, comprising:
a fragmentation step of fragmenting an at least partially crosslinked extracellular matrix component in an aqueous medium.

[11] The method according to (10), comprising, before the fragmentation step, a crosslinking step of heating an extracellular matrix component to crosslink at least a part of the extracellular matrix component.

[12] The method according to (11), wherein a heating temperature in the crosslinking step is 100°C or more.

[13] The method according to any one of (10) to (12), comprising, after the fragmentation step, a drying step of drying a fragmented extracellular matrix component.

**Effects of the Invention**

According to the present invention, an extracellular-matrix-containing composition excellent in dispersibility after dry storage, a method for producing the extracellular-matrix-containing composition, and a three-dimensional tissue construct and three-dimensional tissue construct formation agent each comprising the extracellular-matrix-containing composition can be provided. The extracellular-matrix-containing composition of the present invention is redispersible even after being freeze-dried in a fragmented state, and hence storable for a long period of time.

**Brief Description of Drawings**

[Figure 1] Figure 1 shows a photograph of a fragmented crosslinked collagen component before freeze-drying.
[Figure 2] Figure 2 shows a photograph of a fragmented crosslinked collagen component after freeze-drying.
[Figure 3] Figure 3 shows photographs of a fragmented non-crosslinked collagen component before and after freeze-drying.
[Figure 4] Figure 4(A) shows a graph representing results of measurement of crosslinking percentage as determined with a TNBS method, and Figure 4(B) shows a graph representing the variation of crosslinking percentage depending on crosslinking time as determined with a TNBS method.
[Figure 5] Figure 5 shows a diagram representing a method for producing a three-dimensional tissue construct and a three-dimensional tissue construct comprising a fragmented crosslinked collagen component.
[Figure 6] Figures 6(A) and 6(B) respectively show a photograph and graph demonstrating results of water-dispersion stability in a fragmentation step.
[Figure 7] Figure 7 shows photographs demonstrating results of fluorescence imaging of a three-dimensional tissue construct constructed with a fragmented non-crosslinked collagen component and that constructed with a fragmented crosslinked collagen component.
[Figure 8] Figure 8 shows photographs demonstrating results of observation of a fragmented crosslinked collagen component produced with a stirrer-type homogenizer and that produced with an ultrasonic homogenizer.
[Figure 9] Figure 9 shows a graph and photographs demonstrating results of confirmation of dispersibility after freeze-drying.

**Embodiments for Carrying Out the Invention**

Hereinafter, modes for implementing the present invention will be described in detail. However, the present invention is not limited to the following embodiments.

<Extracellular-Matrix-Containing Composition>

The extracellular-matrix-containing composition according to the present embodiment comprises: a fragmented extracellular matrix component, wherein at least a part of the fragmented extracellular matrix component is crosslinked.
The extracellular-matrix-containing composition according to the present embodiment is excellent in dispersibility after dry storage (redispersibility). Because the excellent redispersibility allows easy preparation of a dispersion with a homogeneous concentration, the extracellular-matrix-containing composition according to the present embodiment can be preferably used as a scaffold or the like for formation of three-dimensional tissue constructs.
The extracellular-matrix-containing composition at least contains a fragmented and crosslinked extracellular matrix component. The extracellular-matrix-containing composition may contain a fragmented and non-crosslinked extracellular matrix component, and may contain an at least partially crosslinked and non-fragmented extracellular matrix component.
The extracellular matrix component, formed of multiple extracellular matrix molecules, is an assembly of extracellular matrix molecules. Extracellular matrix molecules refer to substances present out of cells in living organisms. Any substance as an extracellular matrix molecule may be used unless an adverse effect is caused on the growth of cells and formation of a cell assembly. Examples of extracellular matrix molecules include, but are not limited to, collagen, laminin, fibronectin, vitronectin, elastin, tenascin, entactin, fibrillin, and proteoglycan. One of these extracellular matrix components may be used singly, and any combination of them may be used. Modified products and variants of the above-mentioned extracellular matrix molecules are acceptable unless an adverse effect is caused on the growth of cells and formation of a cell assembly.
Examples of collagen include fibrillar collagen and non-fibrillar collagen. Fibrillar collagen refers to collagen that serves as a main component of collagen fibers, and specific examples thereof include type I collagen, type II collagen, and type III collagen. Examples of non-fibrillar collagen include type IV collagen.
Examples of proteoglycan include, but are not limited to, chondroitin sulfate proteoglycan, heparan sulfate proteoglycan, keratan sulfate proteoglycan, and dermatan sulfate proteoglycan.
The extracellular matrix component may contain at least one selected from the group consisting of collagen, laminin, and fibronectin, and it is preferable that the extracellular matrix component contain collagen. The collagen is preferably fibrillar collagen, and more preferably type I collagen. Commercially available collagen may be used for the fibrillar collagen, and specific examples thereof include a freeze-dried product of porcine skin collagen type I produced by NH Foods Ltd. It is preferable that the collagen be atelocollagen, a collagen removed of telopeptide. Atelocollagen can be obtained, for example, through pepsin treatment of tropocollagen.

The extracellular matrix component may be an animal-derived extracellular matrix component.

The animal species from which the extracellular matrix component is derived may be, for example, a mammalian species, an avian species, a reptilian species, or a fish species, and it is preferable that the animal species from which the extracellular matrix component is derived be a mammalian species. Examples of the animal species from which the extracellular matrix component is derived include, but are not limited to, humans, pigs, and bovines. The animal species from which the extracellular matrix component is derived may be a mammalian species, and may be a pig. For the extracellular matrix component, a component derived from one animal may be used, and components derived from a plurality of animals may be used in combination. The animal species from which the extracellular matrix component is derived may be the same as or different from the origin of cells for formation of a three-dimensional tissue.

Fragmented extracellular matrix component is a component finely fragmented by applying physical force to the above-described extracellular matrix component. It is preferable that the fragmented extracellular matrix component be a fibrillated extracellular matrix component obtained by fibrillating the extracellular matrix component without cleaving bonds of extracellular matrix molecules. If the fragmented extracellular matrix component is a fibrillated extracellular matrix component, the fragmented extracellular matrix component can be more effectively used as a scaffold material.

The manner of fragmenting the extracellular matrix component is not limited to a particular method. For example, the extracellular matrix component may be fragmented (or fibrillated) by applying physical force with an ultrasonic homogenizer, a stirrer-type homogenizer, a high-pressure homogenizer, or the like. In using a stirrer-type homogenizer, the extracellular matrix component may be directly homogenized, or homogenized in an aqueous medium such as saline. The fragmented extracellular matrix component can be obtained in millimeter-size or nanometer-size by adjusting the duration of homogenization, the number of homogenizing operations, and so forth. Alternatively, the fragmented extracellular matrix component can be obtained by fragmenting through repetitive freezing and thawing.

It is preferable that the fragmented extracellular matrix component contain a fragmented collagen component. If the fragmented collagen component is dispersed in an aqueous medium, it becomes easy for the fragmented collagen component to come into contact with cells in the aqueous medium, which can facilitate formation of a three-dimensional tissue construct. It is preferable that the fragmented collagen component be a fibrillated collagen component.

The fragmented extracellular matrix component may be naturally-occurring. The fragmented extracellular matrix component that is naturally-occurring is a fragmented product of a natural extracellular matrix component, and components obtained by modifying the structure of a natural extracellular matrix molecule with chemical treatment are not included in the category of the fragmented extracellular matrix component that is naturally-occurring. Examples of the chemical treatment include hydrolysis with alkali treatment.

Examples of the shape of the fragmented extracellular matrix component include fibrillar shapes. A fibrillar shape refers to a shape composed of a filamentous extracellular matrix component or a shape composed through crosslinking of a filamentous extracellular matrix component. For example, it is preferable that the fragmented collagen component retain the triple helix structure (fibrillar shape) derived from collagen. It is preferable for more excellent redispersibility that at least a part of the fragmented extracellular matrix component be fibrillar.

In one embodiment, it is preferable that the average length of the fragmented extracellular matrix component be 100 nm to 400 $\mu$m, and the average length of the fragmented extracellular matrix component is more preferably 5 $\mu$m to 400 $\mu$m, 10 $\mu$m to 400 $\mu$m, 22 $\mu$m to 400 $\mu$m, or 100 $\mu$m to 400 $\mu$m, because a thick tissue tends to form. In another embodiment, for tendency of stable tissue formation and more excellent redispersibility, the average length of the fragmented extracellular matrix component may be 100 $\mu$m or less, and is preferably 50 $\mu$m or less, more preferably 30 $\mu$m or less, and even more preferably 25 $\mu$m or less or 20 $\mu$m or less, and may be 15 $\mu$m or less, 10 $\mu$m or less, or 1 $\mu$m or less, and may be 100 nm or more. It is preferable that the average length of most of all the fragmented extracellular matrix component be within the above numerical range. Specifically, it is preferable that the average length of 95% of all the fragmented extracellular matrix component be within the above numerical range. It is preferable that the fragmented extracellular matrix component be a fragmented collagen component whose average length is within the above range.

It is preferable that the average diameter of the fragmented extracellular matrix component be 50 nm to 30 $\mu$m, it is more preferable that the average diameter of the extracellular matrix component be 4 $\mu$m to 30 $\mu$m, and it is even more preferable that the average diameter of the extracellular matrix component be 20 $\mu$m to 30 $\mu$m. It is preferable that the fragmented extracellular matrix component be a fragmented collagen component whose average diameter is within the above range.

The above-described ranges of average length and average diameter are optimized ranges from the viewpoint of tissue formation, and hence it is desirable that the average length or average diameter of the fragmented extracellular matrix component fall within the above ranges of average length and average diameter in the stage that the fragmented extracellular matrix component is resuspended in an aqueous medium for tissue formation after a drying step described later.

The average length and average diameter of the fragmented extracellular matrix component can be determined through measurement of individual parts of the fragmented extracellular matrix component with an optical microscope and subsequent image analysis. Herein, "average length" refers to an average value of lengths in the longitudinal direction of a sample under measurement, and "average diameter" refers to an average value of lengths in the direction perpendicular to the longitudinal direction of a sample under measurement.

In the extracellular-matrix-containing composition according to the present embodiment, at least a part of the fragmented extracellular matrix component is crosslinked. The extracellular matrix component may be intramolecularly or intermolecularly crosslinked in or among extracellular matrix molecules constituting the extracellular matrix component.

Examples of crosslinking methods include, but are not limited to, physical crosslinking with application of heat, an ultraviolet ray, radiation, and chemical crosslinking with a crosslinking agent, enzymatic reaction. Physical crosslinking with application of heat is preferred to avoid addition of artificial factors to the extracellular matrix component as best as possible. The crosslinking (physical crosslinking and chemical crosslinking) may be crosslinking via covalent bonds.

In the case that the extracellular matrix component contains a collagen component, crosslinking may be formed among collagen molecules (triple helix structure), or among collagen fine fibers formed of collagen molecules. It is preferable that the crosslinking be crosslinking caused by heat (thermal crosslinking). Thermal crosslinking can be performed, for example, through heat treatment under reduced pressure with a vacuum pump. In the case that thermal crosslinking of a collagen component is performed, the extracellular matrix component may be crosslinked through the phenomenon that an amino group in a collagen molecule forms a peptide bond (-NH-CO-) with a carboxy group in the same or another collagen molecule.

Alternatively, the extracellular matrix component can be crosslinked by using a crosslinking agent. The crosslinking agent may be, for example, one capable of crosslinking a carboxyl group and an amino group or one capable of crosslinking amino groups. It is preferable for economic efficiency, safety, and operability that the crosslinking agent be, for example, an aldehyde-, carbodiimide-, epoxide-, or imidazole-type crosslinking agent, and specific examples of the crosslinking agent include glutaraldehyde, and water-soluble carbodiimides such as 1-ethyl-3-(3-dimethylaminopropyl)carbodiimide hydrochloride and 1-cyclohexyl-3-(2-morpholinyl-4-ethyl)carbodiimide sulfonate.

The crosslinking percentage may be crosslinking percentage measured with a TNBS 2,4,6-trinitrobenzene sulfonic acid) method. Crosslinking percentage as determined with a TNBS method refers to the fraction of amino groups used for crosslinking among the amino groups that the extracellular matrix component has. Crosslinking percentage as determined with a TNBS method can be quantified, for example, on the basis of a TNBS method described in Non Patent Literature 2.

The crosslinking percentage as determined with a TNBS method may be 1% or more, 2% or more, 4% or more, 8% or more, or 12% or more, and may be 30% or less, 20% or less, or 15% or less. By virtue of the configuration that the crosslinking percentage as determined with a TNBS method is within the range, extracellular matrix molecules can be dispersed to an appropriate degree, and the redispersibility after dry storage is satisfactory. In the case that thermal crosslinking is performed, for example, the crosslinking percentage as determined with a TNBS method tends to be higher as the temperature in heat treatment is increased.

In the case that the extracellular matrix component contains a collagen component, it is preferable that the crosslinking percentage as determined with a TNBS method be in the above range.

The crosslinking percentage may be calculated through quantifying carboxyl groups. In the case of a water-insoluble extracellular matrix component, for example, the crosslinking percentage may be quantified with a TBO (toluidine blue O) method.

The form of the extracellular-matrix-containing composition may be solid or powdery, and is preferably powdery because weighing is likely to be easy. The extracellular-matrix-containing composition may be free of moisture. The moisture in the extracellular-matrix-containing composition can be removed, for example, by using a freeze-drying method. Being free of moisture does not mean being completely free of water molecules, but means being free of moisture to such a degree that drying techniques such as a freeze-drying method can achieve in common sense.

The extracellular-matrix-containing composition according to one embodiment is dispersible in aqueous media. The "aqueous medium" refers to a liquid whose essential constituent component is water. The aqueous medium is not limited to a particular aqueous medium, as long as the aqueous medium allows the extracellular matrix component to stably exist therein. Examples of the aqueous medium include, but are not limited to, saline such as phosphate-buffered saline (PBS), and liquid culture media such as a Dulbecco's Modified Eagle's Medium (DMEM) and a liquid culture medium specialized for vascular endothelial cells (Endothelial Cell Growth Medium 2 (EGM2)).

Whether to be dispersible in aqueous media is determined, for example, by using the following method: 50 mg of the extracellular-matrix-containing composition is added to 5 mL of ultrapure water, and if the extracellular-matrix-containing composition is successfully dispersed in the ultrapure water (if agglomeration, precipitation, or the like is not caused), the extracellular-matrix-containing composition can be determined to be dispersible in aqueous media. The temperature in dispersing the extracellular-matrix-containing composition in ultrapure water may be a temperature equal to or less than culture temperature (e.g., 37°C), or room temperature. A dispersed state refers to such a state that the occurrence of agglomeration, precipitation, or the like is not found by visual observation. Alternatively, whether to be dispersible can be determined, for example, through measurement of transmittance for light with a wavelength of 500 nm. If transmittance for light with a wavelength of 500 nm is 50% or less, preferably 35% or less, more preferably 30% or less, even more preferably 20% or less, further preferably 15% or less, and furthermore preferably 12% or less, determination can be made as being dispersible. Transmittance may be measured for an aqueous solution consisting of the extracellular-matrix-containing composition and water (e.g., ultrapure water) as a sample for measurement. The content of the extracellular-matrix-containing composition in the sample for measurement may be 0.5% by mass based on the total mass of the aqueous solution consisting of the extracellular-matrix-containing composition and water. Transmittance may be measured immediately after adding the extracellular-matrix-containing composition to ultrapure water. Measurement of transmittance can be performed, for example, by measuring transmittance for light with a wavelength of 500 nm with use of an ultraviolet/visible/near-infrared spectrophotometer. Specifically, transmittance can be measured by using a method described later in Examples.

It is preferable that the pH of the aqueous medium be in such a range that an adverse effect is not caused on the growth of cells and formation of a cell assembly. To mitigate burdens on cells when the aqueous solution is loaded into the cells, the pH of the aqueous medium may be, for example, 7.0 or more, and may be 8.0 or less. Specifically, the pH of the aqueous medium may be 7.0, 7.1, 7.2, 7.3, 7.4, 7.5, 7.6, 7.7, 7.8, 7.9, or 8.0. It is preferable that the aqueous medium has buffer capacity in the above pH range, and the aqueous medium is more preferably a liquid culture medium. The liquid culture medium is not limited to a particular liquid culture medium, and a preferred culture medium may be selected according to the type of cells to be cultured. Examples of such culture media include an Eagle's MEM, a DMEM, a Modified Eagle's Medium (MEM), Minimum Essential Medium, an RPMI, and a GlutaMax Medium. The culture medium may be a medium with serum, or a serum-free medium. Further, the liquid culture medium may be a mixed culture medium obtained by mixing two or more culture media.

<Method for Producing Extracellular-Matrix-Containing Composition>

The extracellular-matrix-containing composition may be produced by crosslinking the extracellular matrix component and then fragmenting the resultant, or produced by fragmenting the extracellular matrix component and then crosslinking the resultant. It is preferred to produce the extracellular-matrix-containing composition according to the present embodiment by crosslinking extracellular matrix molecules and then fragmenting the resultant. Thereby, the redispersibility after dry storage is enhanced, which makes it easy to mix with cells, and thus makes it easy to form a three-dimensional tissue construct.

As an example of methods for producing an extracellular-matrix-containing composition, a method for producing an extracellular-matrix-containing composition by crosslinking extracellular matrix molecules and then fragmenting the resultant will be described in the following.

The method for producing an extracellular-matrix-containing composition according to one embodiment comprises: a fragmentation step of fragmenting an at least partially crosslinked extracellular matrix component in an aqueous medium.

The manner of fragmenting an at least partially crosslinked extracellular matrix component may be the same as the method exemplified above. The aqueous medium may be the same as the above-described aqueous medium.

The production method according to the present embodiment may comprise, before the fragmentation step, a crosslinking step of heating an extracellular matrix component to crosslink at least a part of the extracellular matrix component.

In the crosslinking step, the temperature (heating temperature) and time (heating time) in heating the extracellular matrix component may be appropriately set. The heating temperature in the crosslinking step may be, for example, 100°C or more and may be 200°C or less. The heating temperature may be specifically, for example, any of 100°C, 110°C, 120°C, 130°C, 140°C, 150°C, 160°C, 170°C, 180°C, 190°C, 200°C, and 220°C. The heating time (time to retain at the heating temperature) may be appropriately set in view of the heating temperature. In heating at 100°C to 220°C, for example, the heating time may be 6 hours or more and 72 hours or less, and is more preferably 24 hours or more and 48 hours or less. In the crosslinking step, heating may be performed in the absence of a solvent, and heating may be performed under reduced pressure.

The production method according to the present embodiment may comprise, after the fragmentation step, a drying step of drying a fragmented extracellular matrix component.

In the drying step, a fragmented extracellular matrix component is dried. Drying may be performed, for example, with a freeze-drying method. By performing the drying step after the fragmentation step, the aqueous medium is removed from the solution containing the fragmented extracellular matrix component and the aqueous medium. The situation that the aqueous medium is removed does not mean that completely no moisture is attached in the fragmented extracellular matrix component, but means that moisture is not attached to such a degree that the above-described common drying technique can achieve in common sense.

The extracellular-matrix-containing composition can be preferably used as a scaffold for formation of three-dimensional tissue constructs. Accordingly, the extracellular-matrix-containing composition is preferably used for application of forming a three-dimensional tissue construct.

<Three-Dimensional Tissue Construct Formation Agent>

The extracellular-matrix-containing composition is preferred as a scaffold or the like for formation of three-dimensional tissue constructs, and hence a three-dimensional tissue construct formation agent comprising the above-described extracellular-matrix-containing composition is provided in one embodiment of the present invention.

Since the three-dimensional tissue construct formation agent according to the present embodiment comprises the above-described extracellular-matrix-containing composition, thicker three-dimensional tissue constructs can be formed.

The three-dimensional tissue formation agent may be in a state of powder in storage, and it is preferable that the three-dimensional tissue formation agent be in a state of dispersion obtained by dispersing the three-dimensional tissue formation agent in an aqueous medium in the stage of forming a three-dimensional tissue construct.

<Three-Dimensional Tissue Construct>

The three-dimensional tissue construct according to the present embodiment comprises: the above-described extracellular-matrix-containing composition; and a cell. At least a part of cells may be adhering to the extracellular-matrix-containing composition. The "three-dimensional tissue construct" refers to an assembly of cells in which the cells are three-dimensionally disposed via an extracellular matrix component and that is artificially produced through cell culture. The shape of the three-dimensional tissue construct is not limited to a particular shape, and examples thereof include a sheet, a sphere, an ellipsoid, and a cuboid. Here, biological tissues include blood vessels, sweat glands, lymphatic vessels, and sebaceous glands, and their configurations are more complex than that of the three-dimensional tissue construct. Therefore, the three-dimensional tissue construct and biological tissues can be easily distinguished from each other.

The cells are not limited to particular cells, and may be, for example, cells derived from an animal such as a human, a monkey, a dog, a cat, a rabbit, a pig, a bovine, a mouse, or a rat. The site from which the cells are derived is not limited to a particular site, and the cells may be somatic cells derived from, for example, the bone, muscle, internal organ, nerve, brain, bone, skin, or blood, and may be germ cells. Moreover, the cells may be induced pluripotent stem cells (iPS cells) or embryonic stem cells (ES cells), or cultured cells such as primary cultured cells, subcultured cells, and cell line cells. Specific examples of the cells include, but are not limited to, neurons, dendritic cells, immunocytes, vascular endothelial cells (e.g., human umbilical vein endothelial cells (HUVEC)), lymphatic endothelial cells, fibroblasts, colon cancer cells (e.g., human colon cancer cells (HT29)), carcinoma cells such as hepatic carcinoma cells, epithelial cells (e.g., human gingival epithelial cells), keratinocytes, cardiomyocytes (e.g., human-iPS-cell-derived cardiomyocytes (iPS-CM)), hepatocytes, pancreatic islet cells, tissue stem cells, and smooth muscle cells (e.g., aortic smooth muscle cells (Aorta-SMC)). One type of cells may be used singly, and multiple types of cells may be used in combination.

It is preferable that the cells include collagen-secreting cells, which secrete collagen such as fibrillar collagen. Examples of collagen-secreting cells include mesenchymal cells such as fibroblasts, chondrocytes, and osteoblasts, and fibroblasts are preferred. Examples of preferred fibroblasts include normal human dermal fibroblasts (NHDF), normal human cardiac fibroblasts (NHCF), and human gingival fibroblasts (HGF).

In the case that the three-dimensional tissue construct includes collagen-secreting cells as the cells, the three-dimensional tissue construct may contain endogenous collagen. The "endogenous collagen" refers to collagen which collagen-producing cells constituting the three-dimensional tissue construct produce. The endogenous collagen may be fibrillar collagen or non-fibrillar collagen.

In the case that the three-dimensional tissue construct contains collagen-secreting cells as the cells, the three-dimensional tissue construct may contain cells including collagen-secreting cells, the extracellular-matrix-containing composition, and an endogenous collagen component. In this case, at least a part of the cells including collagen-secreting cells may be adhering to the extracellular-matrix-containing composition and/or endogenous collagen component. Conventional three-dimensional tissue constructs have low collagen concentration and high cell density. For this reason, conventional three-dimensional tissue constructs suffer from problems of contraction thereof due to tractive force by cells during or after culture, a tendency to be decomposed by an enzyme which cells produce during or after culture, and so forth. The three-dimensional tissue construct according to one embodiment have higher collagen concentration than conventional ones, and is less likely to undergo contraction and thus is stable.

The three-dimensional tissue construct may include collagen-secreting cells and cells other than collagen-secreting cells as the cells. Examples of cells other than collagen-producing cells include vascular endothelial cells (e.g., human umbilical vein endothelial cells (HUVEC)), cancer cells such as colon cancer cells (e.g., human colon cancer cells (HT29)) and hepatic cancer cells, cardiomyocytes (e.g., human-iPS-cell-derived cardiomyocytes (iPS-CM)), epithelial cells (e.g., human gingival epithelial cells), keratinocytes, lymphatic endothelial cells, neurons, hepatocytes, tissue stem cells, embryonic stem cells, induced pluripotent stem cells, adhesive cells (e.g., immunocytes), and smooth muscle cells (e.g., aortic smooth muscle cells (Aorta-SMC)). Preferably, the cells constituting the above three-dimensional tissue construct further include one or more types of cells selected from the group consisting of vascular endothelial cells, cancer cells, and cardiomyocytes.

The content ratio of collagen in the three-dimensional tissue construct may be 0.01 to 90% by mass based on the above three-dimensional tissue construct (dry weight), and it is preferable that the content ratio of collagen in the three-dimensional tissue construct be 10 to 90% by mass, it is preferable that the content ratio of collagen in the three-dimensional tissue construct be 10 to 80% by mass, it is preferable that the content ratio of collagen in the three-dimensional tissue construct be 10 to 70% by mass, it is preferable that the content ratio of collagen in the three-dimensional tissue construct be 10 to 60% by mass, it is preferable that the content ratio of collagen in the three-dimensional tissue construct be 1 to 50% by mass, it is preferable that the content ratio of collagen in the three-dimensional tissue construct be 10 to 50% by mass, it is more preferable that the content ratio of collagen in the three-dimensional tissue construct be 10 to 30% by mass, and it is more preferable that the content ratio of collagen in the three-dimensional tissue construct be 20 to 30% by mass.

Here, the "collagen in the three-dimensional tissue construct" refers to collagen constituting the three-dimensional tissue construct, and may be endogenous collagen or collagen derived from the fragmented collagen component (exogenous collagen). It follows that in the case that the three-dimensional tissue construct contains an endogenous collagen and a fragmented collagen component, the concentration of the above collagen constituting the three-dimensional tissue construct refers to the total concentration of the endogenous collagen component and the fragmented collagen component. The concentration of the above collagen can be calculated from the volume of the three-dimensional tissue construct obtained and the mass of the decellularized three-dimensional tissue construct.

Examples of methods for quantifying the amount of collagen in the three-dimensional tissue construct include a method of quantifying hydroxyproline as follows. Hydrochloric acid (HCl) is mixed in a lysis solution obtained by lysing the three-dimensional tissue construct; the resultant is incubated at a high temperature for a predetermined time; the temperature is then returned to room temperature; and centrifugation is performed and the resulting supernatant is diluted to a predetermined concentration to prepare a sample. Hydroxyproline standard solution is treated in the same manner as for the sample, and serial dilution is performed to prepare standards. The sample and standards are each subjected to a predetermined treatment with a hydroxyproline assay buffer and detection reagent, and absorbance at 570 nm is measured. The absorbance of the sample is compared with those of the standards to calculate the amount of collagen. Alternatively, a lysis solution obtained by directly suspending and dissolving the three-dimensional tissue construct in hydrochloric acid with a high concentration is centrifuged to collect the supernatant, which may be used for quantification of collagen. The three-dimensional tissue construct to be lysed may be in a state as recovered from culture solution, and may be subjected to dry treatment after recovery and lysed with the liquid components removed. If quantification of collagen is performed after the three-dimensional tissue construct in a state as recovered from culture solution is lysed, however, it is expected that culture medium components which the three-dimensional tissue construct has absorbed and a residual culture medium due to a problem in experimental operations cause variation of measurement values of the weight of the three-dimensional tissue construct, and hence it is preferred to use the weight after drying as a reference in order to stably measure the amount of collagen relative to the weight of the tissue or per unit weight.

More specific examples of methods for quantifying the amount of collagen include the following method.

(Preparation of Sample)

The whole of the three-dimensional tissue construct subjected to freeze-drying treatment is mixed with 6 mol/L HCl, the mixture is incubated in a heat block at 95°C for 20 hours or more, and the temperature is then returned to room temperature. Centrifugation is performed at 13000 g for 10 minutes, and the supernatant of the sample solution is then collected. The supernatant is appropriately diluted with 6 mol/L HCl so that results of measurement described later can fall within the range of a calibration curve, and 200 μL of the resultant is diluted with 100 μL of ultrapure water to prepare a sample. The usage of the sample is 35 μL.

(Preparation of Standards)

Into a screwcap tube, 125 μL of standard solution (1200 μg/mL in acetic acid) and 125 μL of 12 mol/L HCl are added and mixed together, the mixture is incubated in a heat block at 95°C for 20 hours, and the temperature is then returned to room temperature. Centrifugation is performed at 13000 g for 10 minutes, the supernatant is then diluted with ultrapure water to produce 300 μg/mL S1, and S1 is subjected to serial dilution to produce S2 (200 μg/mL), S3 (100 μg/mL), S4 (50 μg/mL), S5 (25 μg/mL), S6 (12.5 μg/mL), and S7 (6.25 μg/mL). Additionally, S8 (0 μg/mL), which consists only of 90 μL of 4mol/L HCl, is prepared.

(Assay)

The standards and the sample each in a volume of 35 $\mu$L are added to a plate (attached to a QuickZyme Total Collagen Assay Kit, QuickZyme Biosciences). To each well, 75 $\mu$L of assay buffer (attached to the kit) is added. The plate is sealed, and incubated at room temperature with shaking for 20 minutes. The plate is unsealed, and 75 $\mu$L of detection reagent (reagent A:B = 30 $\mu$L:45 $\mu$L, attached to the kit) is added to each well. The plate is sealed, and incubated at 60°C for 60 minutes while the solutions are mixed by shaking. The plate is sufficiently cooled on ice, and unsealed and absorbance at 570 nm is measured. The absorbance of the sample is compared with those of the standards to calculate the amount of collagen.

Alternatively, collagen in the three-dimensional tissue construct may be specified with the area ratio or volume ratio. "Specifying with the area ratio or volume ratio" means that, for example, collagen in the three-dimensional tissue construct is made distinguishable from other tissue constituents by using a known staining method (e.g., immunostaining with an anti-collagen antibody, and Masson's trichrome staining) or the like, and then the ratio of regions in which collagen is present to the total of the three-dimensional tissue construct is calculated by using any of visual observation, microscopes, image analysis software, and so forth. In specifying with the area ratio, there is no limitation to which cross-section or surface in the three-dimensional tissue construct is used for specifying the area ratio, and in the case that the three-dimensional tissue construct is a sphere or the like, for example, a cross-sectional view along the generally central portion may be used for specification.

In specifying collagen in the three-dimensional tissue construct with the area ratio, the fraction of area is 0.01 to 99% based on the total area of the three-dimensional tissue construct, and it is preferable that the fraction of area be 1 to 99%, it is preferable that the fraction of area be 5 to 90%, it is preferable that the fraction of area be 7 to 90%, it is preferable that the fraction of area be 20 to 90%, and it is more preferable that the fraction of area be 50 to 90%. "Collagen in the three-dimensional tissue construct" is as described above. In the case that the three-dimensional tissue construct contains exogenous collagen, the fraction of area of collagen constituting the three-dimensional tissue construct refers to the fraction of combined areas of endogenous collagen and exogenous collagen. For example, as the three-dimensional tissue construct obtained is stained with Masson's trichrome, the fraction of area of collagen can be calculated as the fraction of area of collagen stained blue to the total area of a cross-section along a generally central portion of the three-dimensional tissue construct.

It is preferable for the three-dimensional tissue construct that the residue proportion after trypsin treatment with a trypsin concentration of 0.25% at a temperature of 37°C and pH 7.4 for a reaction time of 15 minutes be 70% or more, it is more preferable that the residue proportion be 80% or more, and it is even more preferable that the residue proportion be 90% or more. Such a three-dimensional tissue construct is less likely to undergo decomposition due to an enzyme during or after culture, and thus is stable. The residue proportion can be calculated, for example, from the mass of the three-dimensional tissue construct before and after trypsin treatment.

For the three-dimensional tissue construct, the residue proportion after collagenase treatment with a collagenase concentration of 0.25% at a temperature of 37°C and pH 7.4 for a reaction time of 15 minutes may be 70% or more, it is more preferable that the residue proportion be 80% or more, and it is even more preferable that the residue proportion be 90% or more. Such a three-dimensional tissue construct is less likely to undergo decomposition due to an enzyme during or after culture, and thus is stable.

It is preferable that the thickness of the three-dimensional tissue construct be 10 $\mu$m or more, it is more preferable that the thickness of the three-dimensional tissue construct be 100 $\mu$m or more, and it is even more preferable that the thickness of the three-dimensional tissue construct be 1000 $\mu$m or more. The structure of such a three-dimensional tissue construct is more similar to those of biological tissues, and preferred as an alternative for experimental animals and a material for transplantation. The upper limit of the thickness of the three-dimensional tissue construct is not limited to particular values, and may be, for example, 10 mm or less, 3 mm or less, 2 mm or less, 1.5 mm or less, or 1 mm or less.

Here, "the thickness of the three-dimensional tissue construct" refers to, in the case that the three-dimensional tissue construct is a sheet or cuboid, the distance between both ends in the direction perpendicular to a major surface. In the case that unevenness is present in the major surface, the thickness refers to the distance at the thinnest portion of the major surface.

In the case that the three-dimensional tissue construct is a sphere, the thickness refers to the diameter. Further, in the case that the three-dimensional tissue construct is an ellipsoid, the thickness refers to the minor axis. In the case that the three-dimensional tissue construct is a generally spherical or generally ellipsoidal shape and unevenness is present in the surface, the thickness refers to the shortest distance among those between two points at which a line passing through the center of gravity of the three-dimensional tissue construct and the surface intersect.

<Method for Producing Three-Dimensional Tissue Construct>

The method for producing a three-dimensional tissue construct according to the present embodiment includes:

(1) a step of bringing the above-described extracellular-matrix-containing composition into contact with cells in an aqueous medium (step (1)); and (2) a step of culturing the cells brought into contact with the above-described extracellular-matrix-containing composition (step (2)).

In the method for producing a three-dimensional tissue construct, it is preferable that the cells be cells including collagen-producing cells. By using cells including collagen-secreting cells, a more stable three-dimensional tissue construct in which cells are homogeneously distributed can be obtained. Although details for the mechanism of providing such a three-dimensional tissue construct are unclear, the mechanism is inferred as follows.

In conventional methods of producing a three-dimensional tissue construct with use of a scaffold, it is difficult to distribute cells homogeneously into the inside of a scaffold because cells of interest are injected into a scaffold prepared in advance. In the case that the cells are cells including collagen-producing cells, the cells first come into contact with the surface of the extracellular-matrix-containing composition and adhere to it. Thereafter, the cells by themselves produce a protein constituting an extracellular matrix component (e.g., collagen such as fibrillar collagen). The protein produced comes into contact with the surface of the extracellular-matrix-containing composition and adheres to it to function as a crosslinking agent for the extracellular-matrix-containing composition, and organization of the protein and so forth constituting the extracellular matrix component proceeds in an environment in which the cells are homogeneously present. As a result, a more stable three-dimensional tissue construct in which cells are homogeneously distributed is obtained. It should be understood, however, that the inference does not limit the present invention.

The production methods described in Patent Literatures 1 to 3 include many steps for producing a three-dimensional tissue construct, and require an operation time of about 1 hour. The production method according to the present embodiment enables production of a three-dimensional tissue construct in short operation time. Further, the production method according to the present embodiment enables production of a three-dimensional tissue construct in a simple manner. The production method described in Patent Literature 2 requires at least $10^6$ cells for producing a three-dimensional tissue construct having a thickness of about 1 mm. The production method according to the present embodiment enables production of a large-sized three-dimensional tissue construct having a thickness of 1 mm or more with a relatively small number of cells.

In step (1), the extracellular-matrix-containing composition is brought into contact with cells in an aqueous medium. The manner of bringing the extracellular-matrix-containing composition into contact with cells in an aqueous medium is not limited to a particular method. Examples of the manner of bringing into contact include a method of adding the extracellular-matrix-containing composition to a culture solution containing the cells, a method of adding an aqueous medium and the cells to the extracellular-matrix-containing composition, and a method of adding the extracellular-matrix-containing composition and the cells to an aqueous medium prepared in advance.

In step (1), cells including collagen-producing cells and additional cells other than collagen-producing cells may be used. For the collagen-producing cells and the additional cells other than collagen-producing cells, the corresponding cells described above may be used. Through production of a three-dimensional tissue construct with use of collagen-producing cells and additional cells other than collagen-producing cells in combination, various model tissues can be produced. If NHCF and HUVEC are used, for example, a three-dimensional tissue construct including microvessels in the inside can be obtained. If NHCF and colon cancer cells are used, a model tissue of colon cancer can be obtained. If NHCF and iPS-CM are used, a model tissue of myocardia that exhibit synchronized beating can be obtained.

The concentration of the extracellular-matrix-containing composition in step (1) may be appropriately determined according to the intended shape and thickness of the three-dimensional tissue construct, the size of an incubator, and so forth. For example, the concentration of the extracellular-matrix-containing composition in the aqueous medium in step (1) may be 0.1 to 90% by mass or 1 to 30% by mass.

The quantity of the extracellular-matrix-containing composition in step (1) may be 0.1 to 100 mg or 1 to 50 mg per $1 \times 10^5$ cells.

It is preferable that the mass ratio of the extracellular-matrix-containing composition to the cells (extracellular-matrix-containing composition/cells) in step (1) be 1/1 to 1000/1, it is more preferable that the mass ratio be 9/1 to 900/1, and it is even more preferable that the mass ratio be 10/1 to 500/1.

In the case that collagen-producing cells and additional cells are used in combination, the number ratio of the collagen-producing cells in step (1) to the additional cells (ratio of collagen-producing cells/additional cells in step (1)) may be 9/1 to 99/1, 50/50 to 80/20, 20/80 to 50/50, or 10/90 to 50/50.

A step of precipitating both the extracellular-matrix-containing composition and the cells in the aqueous medium may be further included between step (1) and step (2). By performing such a step, the distribution of the extracellular-matrix-containing composition and the cells in the three-dimensional tissue construct becomes more homogeneous. Specific examples of such methods include, but are not limited to, a method of centrifuging a culture solution containing the extracellular-matrix-containing composition and the cells.

Step (1) may be performed by forming a layer of cells in an aqueous medium, followed by bringing an extracellular-matrix-containing composition into contact with the layer. By forming a layer of cells before bringing into contact with an extracellular-matrix-containing composition, a three-dimensional tissue construct whose lower part has a high cell density can be produced. By forming a layer of cells including collagen-producing cells before bringing into contact with an extracellular-matrix-containing composition, a three-dimensional tissue construct whose lower part has a high cell density of cells including collagen-producing cells can be produced. For some types of cells to be used (e.g., aortic smooth muscle cells), a tissue more similar to the corresponding tissue in a living body can be produced through that method.

After step (2), a step of further bringing into contact with cells and culturing the cells may be included as step (3). These cells may be of the same type as the cells used in step (1), or of different type. In the case that cells to be used in step (1) include cells other than collagen-producing cells, for example, cells to be used in step (3) may include collagen-producing cells. In the case that cells to be used in step (1) include collagen-producing cells, for example, cells to be used in step (3) may include cells other than collagen-producing cells. Both of cells to be used in step (1) and cells to be used in step (3) may include collagen-producing cells, and both of cells to be used in step (1) and cells to be used in step (3) may include cells other than collagen-producing cells. Through step (3), a three-dimensional tissue construct of bilayer structure can be produced. In the case that aortic smooth muscle cells and vascular endothelial cells are used, and in the case that human-skin-derived fibroblasts and human epidermal keratinocytes are used, for example, a tissue more similar to the corresponding tissue in a living body can be produced through that method. In the case that human gingival fibroblasts and gingival epithelial cells are used, for example, a three-dimensional tissue construct of bilayer structure without tissue contraction and tissue cracking can be produced through that method.

The manner of culturing the cells brought into contact with the extracellular-matrix-containing composition is not limited to a particular method, and a preferred culture method may be used for culturing according to the type of cells to be cultured. For example, the culture temperature may be 20°C to 40°C or 30°C to 37°C. The pH of the culture medium may be 6 to 8 or 7.2 to 7.4. The culture period may be 1 day to 2 weeks or 1 week to 2 weeks.

The culture medium is not limited to a particular culture medium, and a preferred culture medium may be selected according to the type of cells to be cultured. Examples of such culture media include an Eagle's MEM, a DMEM, a Modified Eagle' Medium (MEM), Minimum Essential Medium, an RPMI, and a GlutaMax Medium. The culture medium may be a medium with serum, or a serum-free medium. Further, the liquid culture medium may be a mixed culture medium obtained by mixing two or more culture media.

The cell density in the culture medium in step (2) may be appropriately determined according to the intended shape and thickness of the three-dimensional tissue construct, the size of an incubator, and so forth. For example, the cell density in the culture medium in step (2) may be 1 to $10^8$ cells/mL or $10^3$ to $10^7$ cells/mL. The cell density in the culture medium in step (2) may be the same as the cell density in the aqueous medium in step (1).

It is preferable that the contraction rate of the three-dimensional tissue construct during culture be 20% or less, it is more preferable that the contraction rate be 15% or less, and it is even more preferable that the contraction rate be 10% or less. The contraction rate can be calculated, for example, by using the following expression, wherein L1 denotes the length of the longest part of the three-dimensional tissue construct 1 day after culture, and L3 denotes the length of the corresponding part of the three-dimensional tissue construct 3 days after culture.

$$\text{Contraction rate (\%)} = \{(L1\text{-}L3)/L1\} \times 100$$

Through the above-described production method, for example, a three-dimensional tissue construct comprising cells and an extracellular matrix component, wherein the content ratio of collagen is 10% by mass to 90% by mass based on the three-dimensional tissue construct, can be produced.

**Examples**

Hereinafter, the present invention will be specifically described on the basis of Examples; however, the present invention is not limited to them.

(Example 1: Production of Crosslinked Collagen Component)

One hundred milligrams of a freeze-dried product of porcine skin collagen type I produced by NH Foods Ltd. was heated at 200°C under reduced pressure for 24 hours by using a vacuum specimen dryer HD-15H (produced by ISHII LABORATORY WORKS CO., LTD.). This provided an at least partially crosslinked collagen component (crosslinked collagen component) as a dried product. No major change in appearance was found for the collagen after heating at 200°C.

(Example 2: Fragmentation of Crosslinked Collagen Component)

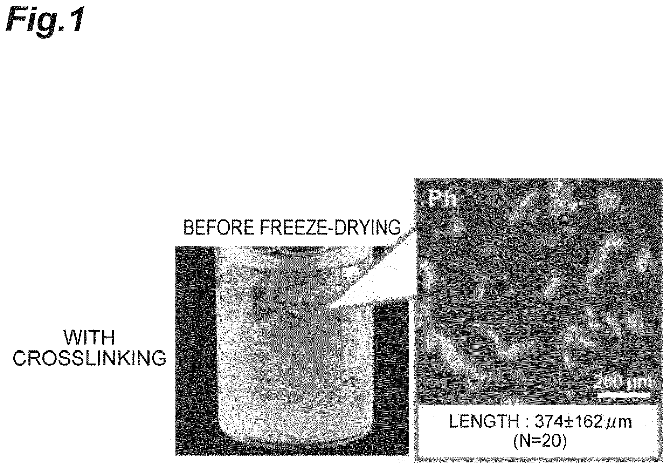

In 5 mL of ultrapure water, 50 mg of the crosslinked collagen component produced in Example 1 was suspended, and the resultant was homogenized by using a stirrer-type homogenizer for 2 minutes to obtain a dispersion containing an at least partially crosslinked fragmented collagen component (fragmented crosslinked collagen component). Figure 1 shows a photograph of the fragmented crosslinked collagen component in the dispersion. The average length (length) of the fragmented crosslinked collagen component obtained was 374 ± 162 μm (number of samples: 20).

(Example 3: Freeze-Drying and Redispersion of Fragmented Crosslinked Collagen Component)

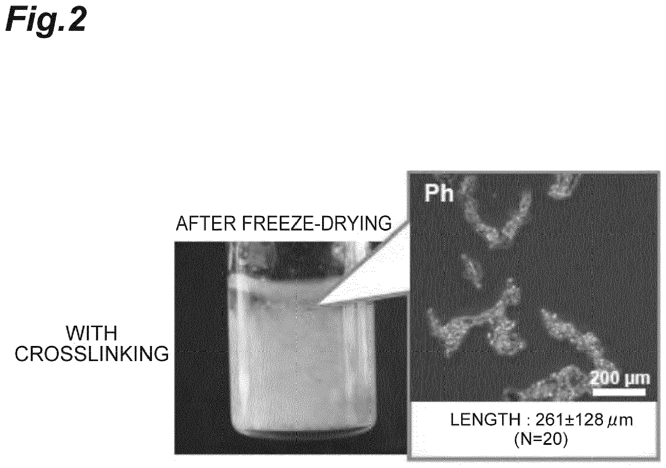

The dispersion containing the fragmented crosslinked collagen component produced in Example 2 was freeze-dried for 3 days to remove moisture. After freeze-drying, the resultant was again suspended in ultrapure water. Figure 2 shows the result. As demonstrated in Figure 2, the fragmented crosslinked collagen component was capable of being resuspended in ultrapure water even after being freeze-dried. The average length (length) of the fragmented crosslinked collagen component after being freeze-dried was 261 ± 128 μm (number of samples: 20).

(Comparative Example 1: Fragmentation of Non-Crosslinked Collagen Component and Redispersibility Thereof After Freeze-Drying)

In 5 mL of ultrapure water, 50 mg of a freeze-dried product of porcine skin collagen type I produced by NH Foods Ltd. without crosslinking treatment was suspended and homogenized for 2 minutes to obtain a dispersion containing a fragmented collagen component that was not crosslinked (fragmented non-crosslinked collagen component). The average length (length) of the fragmented non-crosslinked collagen component was 210 ± 90 μm (number of samples: 20).

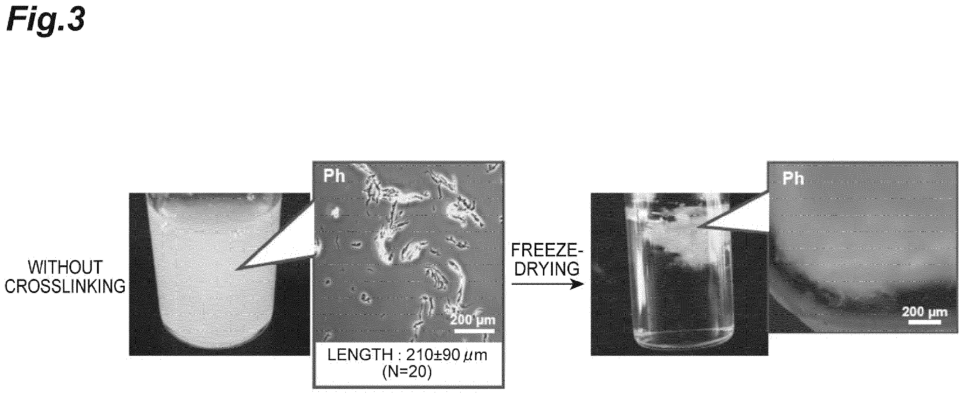

The dispersion containing the fragmented non-crosslinked collagen component was freeze-dried by using a Model FDU-2200 (TOKYO RIKAKIKAI CO., LTD.) for 3 days to remove moisture. After freeze-drying, an attempt was made to again disperse the resultant in ultrapure water. Figure 3 shows the result. As demonstrated in Figure 3, the fragmented non-crosslinked collagen component was being fragmented into a size almost the same as the size of the fragmented crosslinked collagen component and being dispersed in ultrapure water. However, the fragmented non-crosslinked collagen component was not dispersed in ultrapure water even by suspending in ultrapure water after freeze-drying, and a dispersion was not successfully obtained.

(Example 4: Measurement of Crosslinking Percentage of Thermally-Crosslinked Collagen)

The crosslinking percentage of collagen crosslinked at a heating temperature of 200°C was measured by using a TNBS method. The TNBS method was performed in accordance with a method described in Non Patent Literature 2. Specifically, 0.5 mL of 4% $NaHCO_3$ solution (prepared with sodium hydrogen carbonate (FUJIFILM Wako Pure Chemical Corporation, 191-01305) and ultrapure water) and 0.5 mL of 0.5% trinitrobenzenesulfonic acid solution (prepared with sodium 2,4,6-trinitrobenzenesulfonate (NACALAI TESQUE, INC., 35211-44) and ultrapure water) were first added to 4 mg of a thermally-crosslinked collagen component, the resultant was reacted at 40°C for 2 hours, and 1.5 mL of 6 M hydrochloric acid (hydrochloric acid (FUJIFILM Wako Pure Chemical Corporation, 080-01066), 2-fold-diluted), and the resultant was left to stand at 60°C for 90 minutes. Thereafter, 1 mL of the reaction solution was taken out, diluted with 6 mL of ultrapure water, and the absorbance at 345 nm (Abs345nm) was measured by using an ultraviolet/visible/near-infrared spectrophotometer (JASCO Corporation, V-670M). The crosslinking percentage of amino groups was determined with the expression "Crosslinking percentage = 1 - (Abs345nm/Weightcrossiinked)/ (Abs345nm/Weight$_{non-crosslinked}$)".

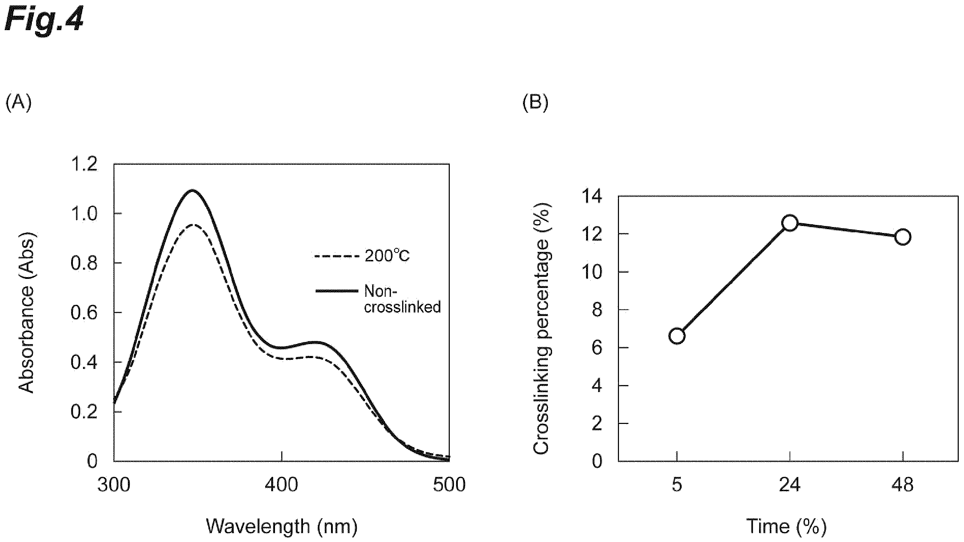

Figure 4(A) shows results of absorption spectrum measurement for crosslinked collagen and non-crosslinked collagen, and Figure 4(B) shows the relation between heating time and crosslinking percentage. No large difference was found between the waveform of the absorption spectrum of the collagen heated at a heating temperature of 200°C and that of the non-heated collagen. That is, it was suggested that structural change that could largely change the physical properties, such as gelatinization, was not caused after heating. The crosslinking percentage when heating was performed at a heating temperature of 200°C for 24 hours was approximately 12%. Further, crosslinking percentages of each sample when the sample was heated at a heating temperature of 200°C for 5 hours to 48 hours were checked. The results showed that no large difference was caused among crosslinking percentages of crosslinked collagen components generated when heating was performed for 24 hours or more.

(Example 5: Tissue Formation with Fragmented Crosslinked Collagen Component)

A freeze-dried product of porcine skin collagen type I produced by NH Foods Ltd. was heated at 200°C for 24 hours in the same manner as in Example 1 to obtain a crosslinked collagen component. The crosslinked collagen component obtained was dispersed in $10\times$ phosphate-buffered saline (X10 PBS), and homogenized by using a stirrer-type homogenizer for 2 minutes to obtain a fragmented crosslinked collagen component. The fragmented crosslinked collagen component was freeze-dried by using a Model FDU-2200 (TOKYO RIKAKIKAI CO., LTD.) for 3 days to remove moisture, and thereafter 8 mg of the fragmented crosslinked collagen component in the freeze-dried state was weighed, and suspended in 300 μL of a mixed culture medium of D-MEM and EBM-2 to obtain a dispersion containing the fragmented crosslinked collagen component.

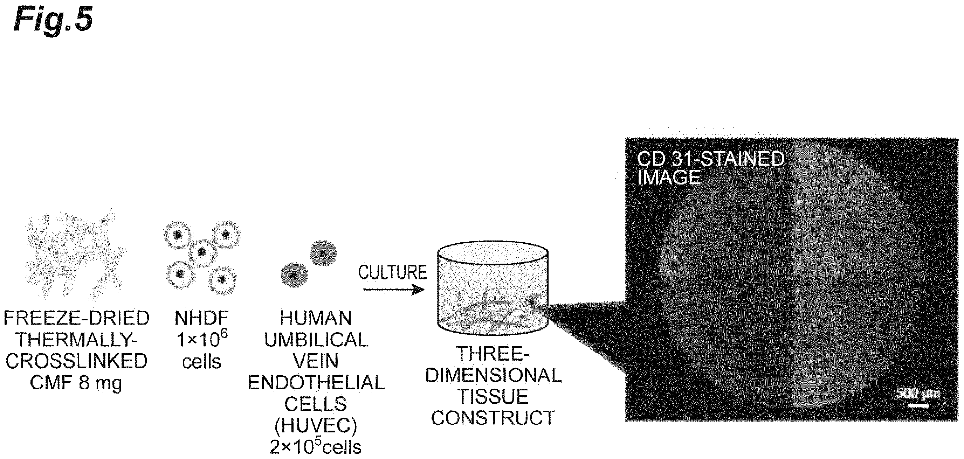

Further, as illustrated in Figure 5, $1.0 \times 10^6$ cells of normal human dermal fibroblasts (NHDF, produced by Lonza) and $2.0 \times 10^5$ cells of human umbilical vein endothelial cells (HUVEC, produced by Lonza) were suspended in the dispersion of the fragmented crosslinked collagen component (freeze-dried thermally-crosslinked CMF), and the suspension was added to a 24-well cell culture insert (produced by Corning Incorporated) and cultured in 2 mL of the mixed culture medium for 1 day. Thereafter, the suspension was transferred into a 6-well plate (produced by IWAKI, AGC TECHNO GLASS Co., Ltd.), and further cultured in 12 mL of the mixed culture medium for 4 days. The three-dimensional tissue construct after culture was subjected to immunofluorescence staining with an anti-CD31 antibody (produced by Dako, M0823) and Alexa647-labeled secondary antibody (produced by Invitrogen, Thermo Fisher Scientific K.K., A-21235) for fluorescence-labeling of blood vessels in the three-dimensional tissue construct. This fluorescence-labeled three-dimensional tissue construct was observed with the confocal quantitative image cytometer CQ1 (produced by Yokogawa Electric Corporation) for the presence or absence of vascular network formation. Figure 5 shows the result. As demonstrated in Figure 5, it was found that a vascular network was formed through formation of the three-dimensional tissue construct with the fragmented crosslinked collagen component.

(Example 6: Effect of Crosslinking Percentage on Water-Dispersion Stability in Fragmentation Step)

The fragmented non-crosslinked collagen component underwent in some cases dissolution of the fragmented collagen component in a solvent while the fragmentation step (homogenization) was continued for a long time. Whether the fragmented crosslinked collagen component had water-dispersion stability in the fragmentation step was examined. Water-dispersion stability refers to such a characteristic that when an extracellular matrix component is included in a solvent containing water as a main component (preferably, ultrapure water), the extracellular matrix component comes into a dispersed state without dissolving therein.

One hundred milligrams of a freeze-dried product of porcine skin collagen type I produced by NH Foods Ltd. was heated at 100°C, 150°C, or 200°C under reduced pressure for 24 hours (crosslinking step). This provided crosslinked collagen components. In 5 mL of ultrapure water, 50 mg of each crosslinked collagen component obtained was suspended, and homogenized by using a stirrer-type homogenizer for 2 minutes to obtain dispersions containing a fragmented crosslinked collagen component (fragmentation step).

The crosslinking percentages of the crosslinked collagen components prepared at a heating temperature of 100°C, 150°C, and 200°C as determined with the TNBS method were 3%, 4%, and 12%, respectively.

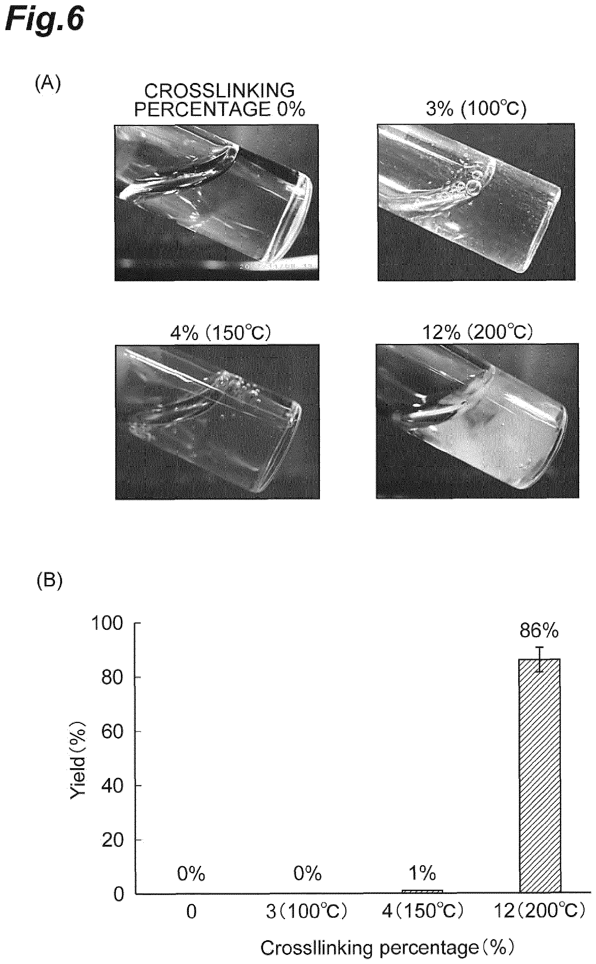

Figure 6(A) shows photographs at a time point after each of the fragmented crosslinked collagen components with a crosslinking percentage of 3%, 4%, or 12% as determined with the TNBS method was included in ultrapure water to reach a concentration of 1% by mass and retained at 4°C for 24 hours. Figure 6(B) shows results of measurement of recovery rates for various collagen components. It was demonstrated that if the crosslinking percentage of a collagen component as determined with the TNBS method is increased, the collagen component is poorly dissolved in water in the fragmentation step and it is easier to recover the collagen component after the fragmentation step. Thus, it was demonstrated that increase in crosslinking percentage as determined with the TNBS method leads to enhanced production efficiency.

(Example 7: Construction of Three-Dimensional Tissue Construct with Fragmented Crosslinked Collagen)

In the same manner as in Example 5, 8 mg of the fragmented crosslinked collagen component in a state of a freeze-dried product obtained (cCMF) was weighed, and suspended in 300 μL of a mixed culture medium of D-MEM and EBM-2 to obtain a dispersion containing the fragmented crosslinked collagen component (cCMF). In the same manner, a dispersion containing a fragmented non-crosslinked collagen component (CMF) was obtained.

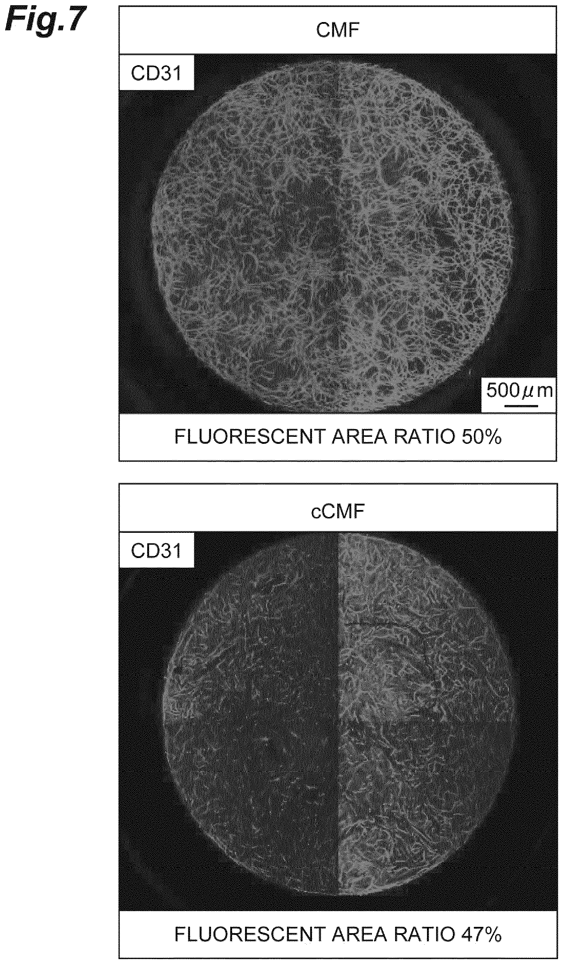

In each of the dispersion containing the fragmented crosslinked collagen component (cCMF) and that containing the fragmented non-crosslinked collagen component (CMF), $1.0 \times 10^6$ cells of normal human dermal fibroblasts (NHDF, produced by Lonza) and $5.0 \times 10^5$ cells of human umbilical vein endothelial cells (HUVEC, produced by Lonza) were suspended, and the suspension was added to a 24-well cell culture insert (produced by Corning Incorporated) and cultured in 2 mL of the mixed culture medium for 1 day. Thereafter, the suspension was transferred into a 6-well plate (produced by IWAKI, AGC TECHNO GLASS Co., Ltd.), and further cultured in 12 mL of the mixed culture medium for 4 days. Each three-dimensional tissue construct after culture was subjected to immunofluorescence staining with an anti-CD31 antibody (produced by Dako, M0823) and Alexa647-labeled secondary antibody (produced by Invitrogen, Thermo Fisher Scientific K.K., A-21235) for fluorescence-labeling of blood vessels in the three-dimensional tissue construct. These fluorescence-labeled three-dimensional tissue constructs were observed with the confocal quantitative image cytometer CQ1 (produced by Yokogawa Electric Corporation) for vascular network formation. Figure 7 shows the results. As demonstrated in Figure 7, it was revealed that the presence or absence of crosslinking did not affect the activity of cells in construction of a three-dimensional tissue construct.

(Example 8: Production of Fragmented Crosslinked Collagen with Ultrasonication)

In 5 mL of ultrapure water, 50 mg of the crosslinked collagen component produced in Example 1 was suspended. An operation to ultrasonicate a tube containing the suspension on ice by using an ultrasonic homogenizer (Sonics & Materials, Inc., VC 50) for 20 seconds and then leave the tube to stand for 10 seconds was repeated 100 times. This provided a dispersion containing an at least partially crosslinked fragmented collagen component (fragmented crosslinked collagen component).

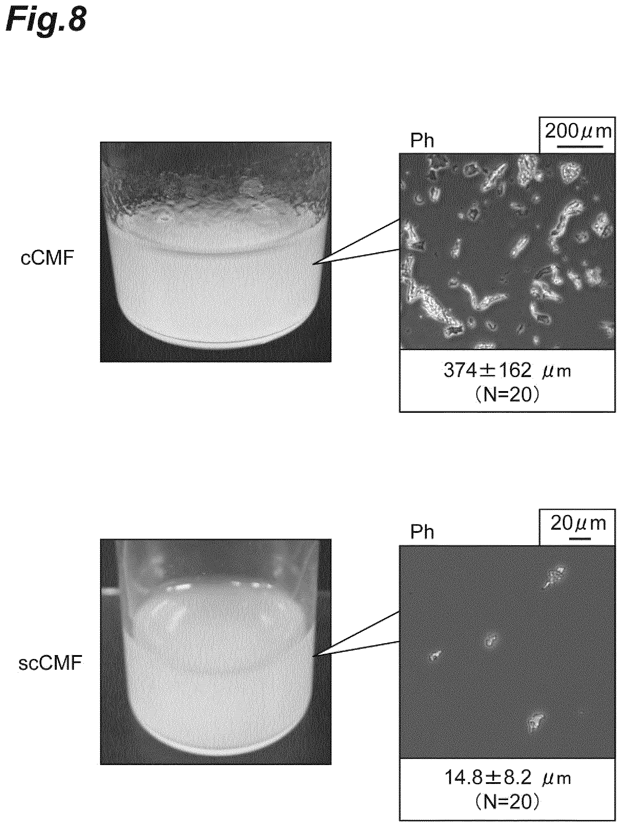

Figure 8 shows photographs and microphotographs demonstrating the appearance of the fragmented crosslinked collagen component produced with a stirrer-type homogenizer (cCMF) and that of the fragmented crosslinked collagen component produced with an ultrasonic homogenizer (ultrasonication) (scCMF). The average length of the fragmented crosslinked collagen component produced with ultrasonication (scCMF) was $14.8 \pm 8.2$ $\mu$m (N = 20). It was demonstrated that a fragmented crosslinked collagen of smaller size is obtained through ultrasonication.

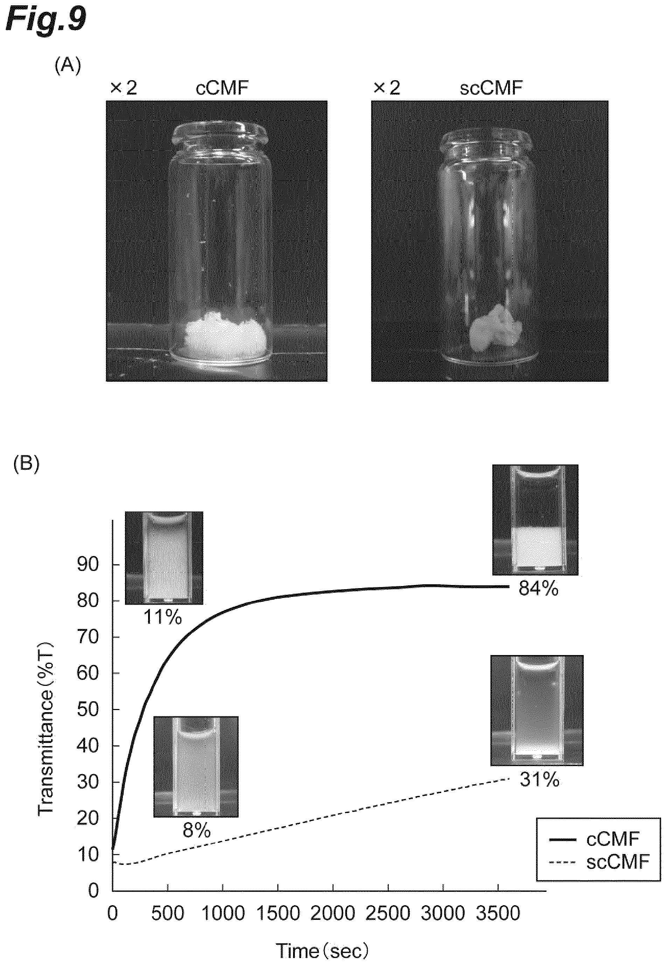

The dispersion containing cCMF and that containing scCMF were frozen, and dried for 3 days to remove moisture. This provided cCMF and scCMF as dried products (Figure 9(A)).
After freeze-drying, each of cCMF and scCMF were again suspended in 2 mL of ultrapure water at a temperature of 20°C to reach a concentration of 0.5% by mass. The state of each suspension was observed for 1 hour as the starting time point of suspension after freeze-drying was defined as 0 s. Figure 9(B) shows the results. As demonstrated in Figure 9(B), transmittance for light with a wavelength of 500 nm at 0 s, the starting time point of suspension after freeze-drying, was 11% for the ultrapure water with cCMF suspended therein, and 8% for the ultrapure water with scCMF suspended therein, and cCMF and scCMF were both confirmed to be redispersible after freeze-drying. In the case with scCMF, the dispersed state was retained for a longer period of time. This revealed that decrease in the size (average length) of a fragmented thermally-crosslinked collagen component (e.g., decrease to 50 $\mu$m or less, preferably to 20 $\mu$m or less) facilitates retention of high dispersibility. The measurement of transmittance was performed by measuring transmittance for light with a wavelength of 500 nm with use of a V-670 spectrophotometer (JASCO Corporation).

**Claims**

1. An extracellular-matrix-containing composition comprising:

   a fragmented extracellular matrix component, wherein
   at least a part of the fragmented extracellular matrix component is crosslinked.

2. The extracellular-matrix-containing composition according to claim 1, wherein at least a part of the fragmented extracellular matrix component is fibrillar.

3. The extracellular-matrix-containing composition according to claim 1 or 2, wherein the extracellular-matrix-containing composition is dispersible in an aqueous medium.

4. The extracellular-matrix-containing composition according to any one of claims 1 to 3, wherein an average length of the fragmented extracellular matrix component is 100 nm to 400 $\mu$m.

5. The extracellular-matrix-containing composition according to any one of claims 1 to 4, wherein the fragmented extracellular matrix component comprises a fragmented collagen component.

6. The extracellular-matrix-containing composition according to any one of claims 1 to 5, wherein a crosslinking percentage as determined with a TNBS method is 2% or more.

7. The extracellular-matrix-containing composition according to any one of claims 1 to 6, wherein the extracellular-matrix-containing composition is powdery.

8. A three-dimensional tissue construct formation agent comprising:
the extracellular-matrix-containing composition according to any one of claims 1 to 7.

9. A three-dimensional tissue construct comprising:

the extracellular-matrix-containing composition according to any one of claims 1 to 7; and
a cell.

10. A method for producing an extracellular-matrix-containing composition comprising a fragmented extracellular matrix component, comprising:
a fragmentation step of fragmenting an at least partially crosslinked extracellular matrix component in an aqueous medium.

11. The method according to claim 10, comprising, before the fragmentation step, a crosslinking step of heating an extracellular matrix component to crosslink at least a part of the extracellular matrix component.

12. The method according to claim 11, wherein a heating temperature in the crosslinking step is 100°C or more.

13. The method according to any one of claims 10 to 12, comprising, after the fragmentation step, a drying step of drying a fragmented extracellular matrix component.

## Fig.1

BEFORE FREEZE-DRYING

WITH
CROSSLINKING

Ph

200 μm

LENGTH : 374±162 μm
(N=20)

# Fig.2

AFTER FREEZE-DRYING

WITH CROSSLINKING

Ph

200 μm

LENGTH : 261±128 μm
(N=20)

*Fig.3*

WITHOUT CROSSLINKING

LENGTH : 210±90 μm
(N=20)

Ph

200 μm

FREEZE-DRYING

Ph

200 μm

# *Fig.4*

(A)

(B)

EP 3 786 179 A1

Fig.5

# *Fig.6*

(A)

CROSSLINKING PERCENTAGE 0%

3% (100℃)

4% (150℃)

12% (200℃)

(B)

**Fig.7**

CMF

CD31

500μm

FLUORESCENT AREA RATIO 50%

cCMF

CD31

FLUORESCENT AREA RATIO 47%

## Fig.8

cCMF

Ph          200μm

374±162 μm
(N=20)

scCMF

Ph          20μm

14.8±8.2 μm
(N=20)

## *Fig.9*

(A)

(B)

**EP 3 786 179 A1**

## INTERNATIONAL SEARCH REPORT

| | International application No. |
|---|---|
| | PCT/JP2019/018272 |

**A. CLASSIFICATION OF SUBJECT MATTER**
Int.Cl. C07K14/78(2006.01)i, C12N5/071(2010.01)i

According to International Patent Classification (IPC) or to both national classification and IPC

**B. FIELDS SEARCHED**

Minimum documentation searched (classification system followed by classification symbols)
Int.Cl. C07K14/78, C12N5/071

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2009-131189 A (NIPRO CORP.) 18 June 2009, | 1-9 |
| Y | claims, paragraphs [0004], [0006], [0017]-[0020], examples (Family: none) | 10-13 |
| Y | 加藤菜津子他, コラーゲンマイクロファイバーを用いた三次元がん－間質組織体の構築, 第66回高分子学会年次大会 高分子学会予稿集, 2017, vol. 66, no. 1, 2Pb096, entire text, non-official translation (KATO, Natsuko et al., "Construction of three-dimensional cancer-interstitial tissue using collagen microfiber", Preprints of the 66th SPSJ Annual Meeting) | 10-13 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 July 2019 (29.07.2019) | 13 August 2019 (13.08.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/018272 |

C (Continuation).    DOCUMENTS CONSIDERED TO BE RELEVANT

| Category* | Citation of document, with indication, where appropriate, of the relevant passages | Relevant to claim No. |
|---|---|---|
| Y | 西宏基他，コラーゲンマイクロファイバーを用いた iPS 細胞由来心筋細胞組織の構築，第 66 回高分子学会年次大会 高分子学会予稿集，2017, vol. 66, no. 1, 2Pa097, entire text, non-official translation (NISHI, Koki et al., "Construction of iPS cell-derived cardiomyocyte tissue using collagen microfiber", Preprints of the 66th SPSJ Annual Meeting) | 10-13 |
| Y | 米田美咲他，コラーゲンマイクロファイバーによる高濃度細胞外マトリックスと毛細血管網を有する三次元間質組織体の構築，第 66 回高分子学会年次大会 高分子学会予稿集，2017, vol. 66, no. 2, 2Q10, entire text, non-official translation (YONETA, Misaki et al., "Construction of three-dimensional interstitial tissue with high concentration extracellular matrix and capillary network by collagen microfiber", Preprints of the 66th SPSJ Annual Meeting) | 10-13 |
| Y | 加藤菜津子他，コラーゲンマイクロファイバーを用いた高密度 ECM を有する三次元ヒトがん－間質組織体の構築，第 66 回高分子学会年次大会 高分子学会予稿集，2017, vol. 66, no. 2, 2Q11, entire text, non-official translation (KATO Natsuko et al., "Construction of 3D human cancer-interstitial tissue with high-density ECM using collagen microfiber", Preprints of the 66th SPSJ Annual Meeting) | 10-13 |
| Y | 松崎典弥他，コラーゲンマイクロファイバーを用いた沈殿培養による高密度 ECM を有する 3D－結合組織の構築，第 66 回高分子学会年次大会 高分子学会予稿集，2017, vol. 66, no. 2, 3N02, entire text, non-official translation (MATSUSAKI Michiya et al., "Construction of 3D-connective tissue having high density ECM through sedimentation culture in which collagen microfiber is used", Preprints of the 66th SPSJ Annual Meeting) | 10-13 |
| Y | 西宏基他，コラーゲンマイクロファイバーを用いた iPS 細胞由来心筋細胞線維化モデルの構築，第 66 回高分子学会年次大会高分子学会予稿集，2017, vol. 66, no. 2, 3N03, entire text, non-official translation (NISHI, Koki et al., "Establishment of iPS cell-derived cardiac fibrosis model using collagen microfiber", Preprints of the 66th SPSJ Annual Meeting) | 10-13 |

Form PCT/ISA/210 (continuation of second sheet) (January 2015)

**INTERNATIONAL SEARCH REPORT**

International application No.

PCT/JP2019/018272

C (Continuation). DOCUMENTS CONSIDERED TO BE RELEVANT

| Category* | Citation of document, with indication, where appropriate, of the relevant passages | Relevant to claim No. |
|---|---|---|
| Y | 松崎典弥他, 生体類似の高密度な細胞外マトリックスと毛細血管を有する三次元間質組織体の構築, 日本化学会第97春季年会(2017)講演予稿集, 2017, 3C1-03, entire text, non-official translation (MATSUSAKI, Michiya et al., "Construction of three-dimensional interstitial tissue with biosimilar dense extracellular matrix and capillaries", Lecture preprints of the 97th spring annual conference of the Chemical Society of Japan (2017)) | 10-13 |
| Y | 加藤菜津子他, コラーゲンマイクロファイバーを用いた高密度ECMを有する三次元ヒトがん－間質組織体の構築, 第17回日本再生医療学会総会, March 2018, 0-11-6, entire text, non-official translation (KATO, Natsuko et al., "Construction of 3D human cancer interstitial tissue with high-density ECM using collagen microfiber", The 17th Congress of the Japanese Society for Regenerative Medicine) | 10-13 |
| Y | 西宏基他, コラーゲンマイクロファイバーとiPS細胞由来心筋細胞によるヒト心筋線維化モデルの構築, 第17回日本再生医療学会総会, March 2018, 0-31-4, entire text, non-official translation (NISHI, Koki et al., "Construction of human myocardial fibrosis model using collagen microfiber and iPS cell-derived cardiomyocytes", The 17th Congress of the Japanese Society for Regenerative Medicine) | 10-13 |
| P, X | 中康博他, コラーゲンファイバー架橋のよる物理的安定性の向上と三次元組織構築への応用, 第67回高分子学会年次大会 高分子学会予稿集, May 2018, vol. 67, no. 1, 2H11, entire text, non-official translation (NAKA, Yasuhiro et al., "Improvement of physical stability by collagen fiber cross-linking and application to 3D tissue construction", Preprints of the 67th SPSJ Annual Meeting) | 1-13 |
| P, X | 中康博他, 足場材料のマイクロ化による新規なボトムアップ組織構築法の創製, 第40回日本バイオマテリアル学会大会予稿集, November 2018, p. 421, 2P-050, entire text, non-official translation (NAKA, Yasuhiro et al., "Creation of a new bottom-up tissue construction approach by microfabrication of scaffold materials", Preprints of the 40th conference of Japanese Society for Biomaterials) | 1-13 |

Form PCT/ISA/210 (continuation of second sheet) (January 2015)

**EP 3 786 179 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012115254 A
- WO 2015072164 A
- WO 2016027853 A

**Non-patent literature cited in the description**

- *Nature Biotechnology,* 2005, vol. 23 (7), 879-884
- *Acta Biomaterialia,* 2015, vol. 25, 131-142